# EUROPÄISCHE PATENTSCHRIFT

(11) **EP 4 164 548 B1**
(45) Veröffentlichungstag und Bekanntmachung des Hinweises auf die Patenterteilung: **20.12.2023**
(21) Anmeldenummer: 21730595.2
(22) Anmeldetag: 07.06.2021
(51) Int. Cl.: A61F 2/16

(54) **INJEKTORANORDNUNG FÜR EIN EINFÜHREN EINER INTRAOKULARLINSE UND INJEKTOR**
INJECTOR ASSEMBLY FOR INSERTING AN INTRAOCULAR LENS AND INJECTOR
ENSEMBLE INJECTEUR POUR L'INSERTION D'UNE LENTILLE INTRAOCULAIRE ET INJECTEUR

(30) Priorität: 10.06.2020 DE 102020115482
(43) Veröffentlichungstag der Anmeldung: 19.04.2023
(73) Patentinhaber: Carl Zeiss Meditec AG, 07745 Jena (DE)
(72) Erfinder: KOHLSAAT, Malte, 20099 Hamburg (DE); GÜHRS, Julian, 25451 Quickborn (DE); DAMM, Niklas, 10409 Berlin (DE); JASTRAM, Jakob, 12103 Berlin (DE); RINMAN, Alfred, 20357 Hamburg (DE)
(74) Vertreter: PATERIS Patentanwälte PartmbB
(86) Internationale Anmeldenummer: PCT/EP2021/065177
(87) Internationale Veröffentlichungsnummer: WO 2021/249948

(56) Entgegenhaltungen:
- WO-A1-2012/155887
- WO-A2-2018/006889

## Beschreibung

Die Erfindung betrifft eine Injektoranordnung für ein Einführen einer Intraokularlinse in den Kapselsack eines Auges und einen ophthalmochirurgischen Injektor mit der Injektoranordnung.

Bei einer Kataraktbehandlung eines Auges wird herkömmlich nur ein kleiner Schnitt in die Hornhaut des Auges eingebracht, der so groß ist, dass eine Kanüle eines Injektors durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack des Auges abgesaugt. Danach wird eine Intraokularlinse mittels des Injektors in das Auge eingesetzt. Die Intraokularlinse wird in dem Injektor gefaltet, so dass sie durch die Kanüle passt. Die Kanüle wird durch den Schnitt in den Kapselsack eingeführt und die gefaltete Intraokularlinse wird mittels des Injektors durch die Kanüle in den Kapselsack geschoben, in dem die Intraokularlinse sich entfaltet und somit die ursprüngliche Linse ersetzt. Während die Intraokularlinse gefaltet wird oder während die gefaltete Intraokularlinse in den Kapselsack geschoben wird, kann es zu Komplikationen kommen. Die Komplikationen können beispielsweise dazu führen, dass sich die Intraokularlinse nicht vollständig in dem Kapselsack entfaltet oder dass die Intraokularlinse sogar beschädigt wird.

WO 2018/006889 A2 betrifft ein Magazin für ein Injektorsystem mit einem Faltkörper zum Implantieren einer Intraokularlinse in ein Auge. WO 2012/155887 A1 betrifft ein Injektorsystem zum Implantieren einer Intraokularlinse in ein Auge sowie ein Magazin für das Injektorsystem.

Aufgabe der Erfindung ist es daher, eine Injektoranordnung zu schaffen, mit der eine Wahrscheinlichkeit gering ist, dass Fehler bei einer Benutzung der Injektoranordnung auftreten.

Die Aufgabe wird durch eine Injektoranordnung gemäß dem unabhängigen Anspruch 1 gelöst. Vorteilhafte Weiterbildungen der Erfindung sind Gegenstand der Unteransprüche.

Die erfindungsgemäße Injektoranordnung weist auf: eine Faltkammer, welche eingerichtet ist, eine Intraokularlinse , die einen Optikkörper und zwei Haptiken aufweist, aufzunehmen, einen Halter, einen Faltkeil, der an dem Halter angebracht ist und eingerichtet ist, in einer Einführrichtung via eine Faltkammeröffnung in die Faltkammer eingebracht zu werden und dadurch die Intraokularlinse zu falten, und zwei Gleiter, die jeweils eingerichtet sind, durch ein Verschieben des jeweiligen Gleiters aus einer Ausgangsposition in eine Endposition eine der zwei Haptiken auf den Optikkörper zu verschieben, wobei mindestens einer der zwei Gleiter einen Anschlag aufweist, der eingerichtet ist, in der Ausgangsposition des mindestens einen Gleiters an ein an dem Halter angebrachtes Bauteil anzustoßen, wodurch eine Bewegung des Bauteils blockiert ist und es dadurch dem Faltkeil nicht erlaubt ist, die Intraokularlinse zu falten, und in der Endposition des mindestens einen Gleiters die Bewegung des Bauteils zu erlauben und es dadurch dem Faltkeil zu erlauben, die Intraokularlinse zu falten.

Indem die zwei Haptiken mittels der Gleiter auf den Optikkörper angeordnet werden, werden die Haptiken, wenn die Intraokularlinse von dem Faltkeil gefaltet wird, innerhalb des Optikkörpers angeordnet. Dadurch kann ein fehlerarmes und reproduzierbares Einbringen der Intraokularlinse in den Kapselsack eines Auges und ein fehlerarmes und reproduzierbares Entfalten der Intraokularlinse in dem Kapselsack erreicht werden. Durch das Vorsehen des Anschlags kann vermieden werden, dass der Faltkeil in die Faltkammer eingebracht wird, bevor der mindestens eine Gleiter in die Endposition gebracht wurde und somit die zu dem mindestens einen Gleiter zugehörige Haptik auf den Optikkörper geschoben wurde. Dadurch ist Injektoranordnung besonders fehlerarm.

Es ist bevorzugt, dass die zwei Gleiter jeweils einen Anschlag aufweisen. Dadurch ist die Injektoranordnung noch fehlerärmer.

Es ist bevorzugt, dass die zwei Gleiter an einander abgewandten Enden der Faltkammer angeordnet sind. Dabei ist denkbar, dass die zwei Gleiter an einander abgewandten Enden der Faltkammeröffnung angeordnet sind.

Es ist bevorzugt, dass das Bauteil zumindest teilweise von dem Faltkeil gebildet ist. Es ist besonders bevorzugt, dass das Bauteil vollständig vom dem Faltkeil gebildet ist.

Der Anschlag ist bevorzugt von einem Steg gebildet, der in der Einführrichtung gesehen in der Ausgangsposition mit der Faltkammeröffnung überlappt.

Es ist bevorzugt, dass der Anschlag von einem in Richtung zu dem anderen Gleiter hin von dem Gleiter abstehenden Gleitervorsprung des Gleiters gebildet ist und der Faltkeil eine Faltkeilaussparung aufweist, wobei der Gleitervorsprung eingerichtet ist, wenn in der Endposition der Faltkeil in die Faltkammer eingebracht wird, in der Faltkeilaussparung angeordnet zu sein, wodurch die Bewegung des Faltkeils erlaubt ist.

Es ist bevorzugt, dass der Anschlag von dem Gleiter gebildet ist, der eine Gleiteraussparung aufweist, die in einer dem anderen Gleiter zugewandten Seite des Gleiters eingebracht ist, und der Faltkeil einen Faltkeilvorsprung aufweist, der das Bauteil bildet und eingerichtet ist, wenn in der Endposition der Faltkeil in die Faltkammer eingebracht wird, in der Gleiteraussparung angeordnet zu sein, wodurch die Bewegung des Faltkeils erlaubt ist.

Es ist bevorzugt, dass der Anschlag von einem in Richtung entgegen der Einführrichtung von dem Gleiter vorstehenden Gleitervorsprung des Gleiters gebildet ist, die Injektoranordnung einen an dem Halter angebrachten Vorsprung aufweist und der Gleitervorsprung sowie der Faltkeil eine Aussparung begrenzen, wobei der Gleitervorsprung eingerichtet ist, in der Ausgangsposition an den Faltkeil anzustoßen, wodurch das Bauteil von dem Faltkeil gebildet ist, oder an den Vorsprung anzustoßen, wodurch das Bauteil von dem Gleitervorsprung gebildet ist, und, wenn in der Endposition der Faltkeil in die Faltkammer eingebracht wird, in der Aussparung angeordnet zu sein, wodurch die Bewegung des Faltkeils erlaubt ist.

Das Bauteil ist bevorzugt von einem an dem Halter angebrachten Vorsprung gebildet, der von dem Faltkeil räumlich getrennt ist. Der Anschlag ist bevorzugt von dem Gleiter gebildet. Dabei ist besonders bevorzugt, dass der Anschlag von einem Gleitervorsprung des Gleiters gebildet ist. Es ist zudem denkbar, dass der Gleitervorsprung an einer dem anderen Gleiter abgewandten Seite von dem Gleiter vorsteht.

Es ist bevorzugt, dass die Injektoranordnung die Intraokularlinse aufweist. Dabei ist besonders bevorzugt, dass die Intraokularlinse in der Faltkammer angeordnet ist.

Es ist bevorzugt, dass die zwei Haptiken jeweils C-förmig sind.

Der ophthalmochirurgische Injektor weist die Injektoranordnung auf.

Im Folgenden wird anhand der beigefügten schematischen Zeichnungen die Erfindung näher erläutert.
Figur 1 zeigt eine Seitenansicht einer ersten Ausführungsform der erfindungsgemäßen Injektoranordnung mit Gleitern, die in einer Ausgangsposition sind.
Figur 2 zeigt eine Draufsicht der Injektoranordnung aus Figur 1.
Figur 3 zeigt eine Seitenansicht der ersten Ausführungsform mit den Gleitern, die in einer Endposition sind.
Figur 4 zeigt eine Draufsicht der Injektoranordnung aus Figur 3.
Figur 5 zeigt eine Seitenansicht der ersten Ausführungsform mit den Gleitern in der Ausgangsposition, wobei ein Faltkeil der Injektoranordnung an einen der Gleiter anstößt und somit nicht in eine Faltkammer der Injektoranordnung eingebracht werden kann.
Figur 6 zeigt eine Seitenansicht der ersten Ausführungsform mit den Gleitern in der Endposition, wobei der Faltkeil innerhalb der Faltkammer angeordnet ist.
Figur 7 zeigt eine Draufsicht einer zweiten Ausführungsform der erfindungsgemäßen Injektoranordnung mit Gleitern, die in einer Ausgangsposition sind.
Figur 8 zeigt eine Draufsicht der zweiten Ausführungsform, wobei die Gleiter in einer Endposition sind und ein Faltkeil der Injektoranordnung innerhalb einer Faltkammer der Injektoranordnung angeordnet ist.
Figur 9 zeigt eine Draufsicht einer dritten Ausführungsform der erfindungsgemäßen Injektoranordnung mit Gleitern, die in einer Ausgangsposition sind.
Figur 10 zeigt eine Draufsicht der dritten Ausführungsform, wobei die Gleiter in einer Endposition sind und ein Faltkeil der Injektoranordnung innerhalb einer Faltkammer der Injektoranordnung angeordnet ist.
Figur 11 zeigt eine Seitenansicht einer vierten Ausführungsform der erfindungsgemäßen Intraokularlinse mit Gleitern, die in einer Endposition sind und ein Faltkeil der Injektoranordnung innerhalb einer Faltkammer der Injektoranordnung angeordnet ist.
Figur 12 zeigt eine Draufsicht der vierten Ausführungsform, wobei die Gleiter in einer Ausgangsposition sind.
Figur 13 zeigt eine Draufsicht der vierten Ausführungsform, wobei die Gleiter in der Endposition sind.
Figur 14 zeigt eine perspektivische Ansicht einer fünften Ausführungsform der erfindungsgemäßen Intraokularlinse, wobei Gleiter der Injektoranordnung sowohl in einer Ausgangsposition als auch in einer Endposition dargestellt sind.
Figur 15 zeigt eine Seitenansicht der fünften Ausführungsform, wobei die Gleiter in der Ausgangsposition sind.
Figur 16 zeigt eine Seitenansicht der fünften Ausführungsform, wobei die Gleiter in der Endposition sind und ein Faltkeil der Injektoranordnung innerhalb einer Faltkammer der Injektoranordnung angeordnet ist.
Figur 17 zeigt eine perspektivische Ansicht einer sechsten Ausführungsform der erfindungsgemäßen Intraokularlinse, wobei Gleiter der Intraokularlinse sowohl in einer Ausgangsposition als auch in einer Endposition dargestellt sind.

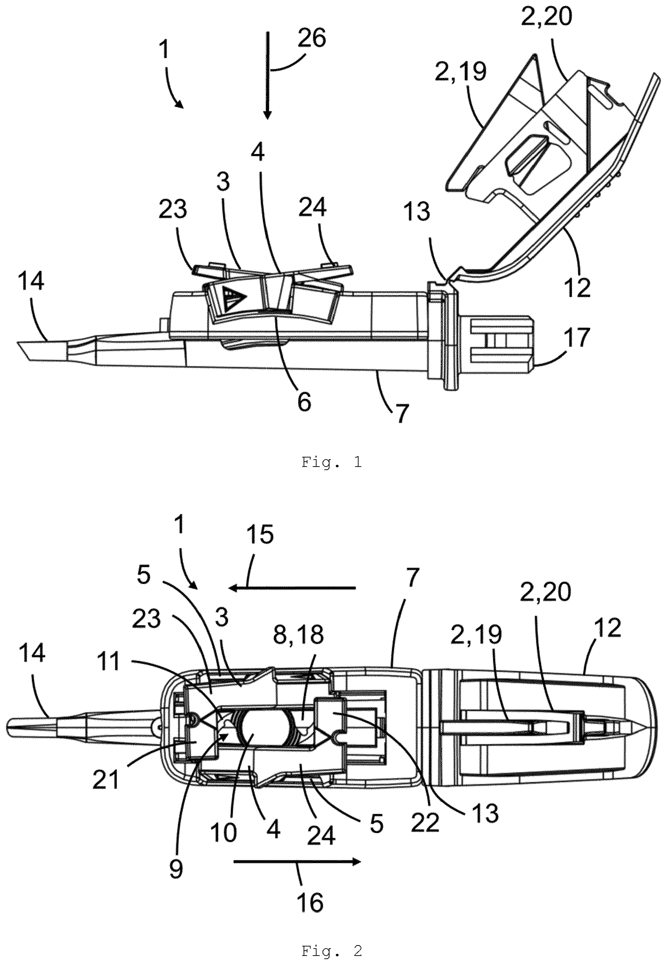

Wie es aus Figuren 1 bis 17 ersichtlich ist, weist eine Injektoranordnung 1 auf: Eine Faltkammer 8, welche eingerichtet ist, eine Intraokularlinse 9, die einen Optikkörper 10 und zwei Haptiken 11 aufweist, aufzunehmen, einen Halter 12, einen Faltkeil 2, der an dem Halter 12 angebracht ist und eingerichtet ist, in einer Einführrichtung 26 via eine Faltkammeröffnung 18 in die Faltkammer 8 eingebracht zu werden und dadurch die Intraokularlinse 9 zu falten, und zwei Gleiter 3, 4, die jeweils eingerichtet sind, durch ein Verschieben des jeweiligen Gleiters 3, 4 aus einer Ausgangsposition in eine Endposition eine der zwei Haptiken 11 auf den Optikkörper 10 zu verschieben, wobei mindestens einer der zwei Gleiter 3, 4 einen Anschlag aufweist, der eingerichtet ist, in der Ausgangsposition des mindestens einen Gleiters 3, 4 an ein an dem Halter 12 angebrachtes Bauteil anzustoßen, wodurch eine Bewegung des Bauteils blockiert ist und es dadurch dem Faltkeil 2 nicht erlaubt ist, die Intraokularlinse 9 zu falten, und in der Endposition des mindestens einen Gleiters 3, 4 die Bewegung des Bauteils zu erlauben und es dadurch dem Faltkeil 2 zu erlauben, die Intraokularlinse 9 zu falten. Die zwei Gleiter 3, 4 werden im Folgenden auch als der erste Gleiter 3 und der zweite Gleiter 4 bezeichnet. Es ist sowohl denkbar, dass lediglich einer der zwei Gleiter 3, 4 den Anschlag aufweist, als auch denkbar, dass die zwei Gleiter 3, 4 jeweils einen Anschlag aufweisen. Dabei ist denkbar, dass der Anschlag des ersten Gleiters 3 symmetrisch zu dem Anschlag des zweiten Gleiters 4 aufgebaut ist. Es ist denkbar, dass die zwei Anschläge eingerichtet sind, an das gleiche Bauteil oder an unterschiedliche Bauteile anzustoßen.

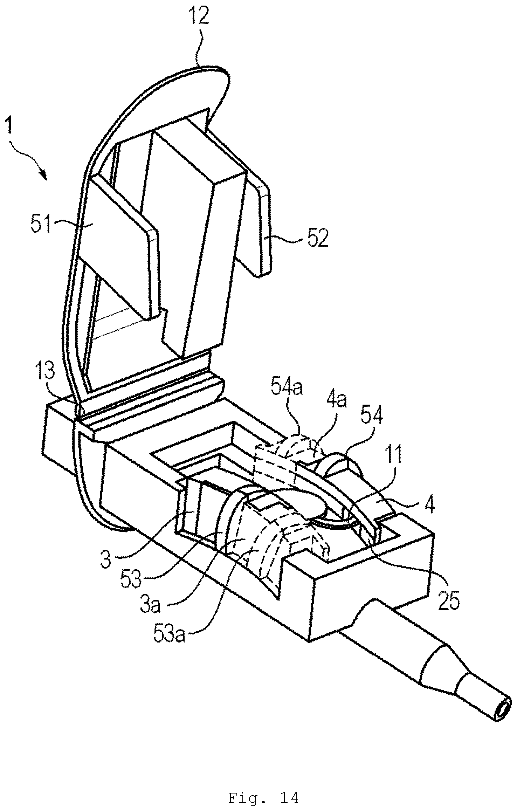

Die Injektoranordnung 1 kann die Intraokularlinse 9 aufweisen. Dabei ist denkbar, dass die Intraokularlinse 1 in der Faltkammer 8 angeordnet ist. Insbesondere Figuren 2 und 14 zeigen, dass die zwei Haptiken 11 jeweils C-förmig sein können.

Insbesondere Figuren 1 und 2 zeigen, dass die zwei Gleiter 3, 4 an einander abgewandten Enden der Faltkammer 8 angeordnet sein können. Die Enden können in einer Richtung senkrecht zu einer Verschieberichtung, in der die Intraokularlinse 9 von der Faltkammer 8 zu einer Kanüle 14 der Injektoranordnung 1 zu verschieben ist, abgewandt voneinander angeordnet sein. Zudem ist erkennbar, dass die Injektoranordnung 1 eine erste Gleiterbahn 5, auf der der erste Gleiter 3 gleitend gelagert ist, und eine zweite Gleiterbahn 6 aufweisen kann, auf der der zweite Gleiter 4 gleitend gelagert ist. Die erste Gleiterbahn 5 und die zweite Gleiterbahn 6 können gekrümmt ausgeführt sein, so dass die Gleiter 3, 4, wenn sie von der Ausgangsposition in die Endposition verschoben werden, einen gekrümmten Weg zurücklegen. Dadurch kann beim Verschieben der Haptiken 11 eine Kollision der Haptiken 11 mit dem Umfangsrand des Optikkörpers 10 vermieden werden. Aus Figur 14 ist ersichtlich, dass die Gleiter 3, 4 jeweils einen Gleiterzapfen 25 aufweisen können, der in Richtung zu der Introkularlinse 9 hin von dem Gleiter 3, 4 absteht. Jeder der Gleiterzapfen 25 ist eingerichtet, jeweils eine der zwei Haptiken 11 zu greifen und auf den Optikkörper 10 zu verschieben, wenn der zu dem Gleiterzapfen 25 zugehörige Gleiter 3, 4 von der Ausgangsposition in die Endposition verschoben wird. Die Gleiterzapfen 25 können von einer Seite des Gleiters 3, 4 abstehen, die dem außerhalb der Faltkammer 8 angeordneten Faltkeil 2 abgewandt angeordnet ist. Dadurch wird erreicht, dass die Haptiken 11 an diejenige Seite des Optikkörpers 10 verschoben werden, die dem Faltkeil 2 zugewandt ist. Dadurch kann erreicht werden, dass die Haptiken 11 innerhalb des Optikkörpers 10 angeordnet werden, wenn der Faltkeil 2 die Intraokularlinse 9 faltet.

Insbesondere aus Figuren 1 und 2 ist ersichtlich, dass die Injektoranordnung 1 einen Injektorkörper 7 aufweisen kann, in dessen Innerem die Faltkammer 8 angeordnet ist. Der Halter 12 kann mittels eines Gelenks 13 an dem Injektorkörper 7 verschwenkbar angebracht sein. Durch ein Verschwenken des Halters 12 um das Gelenk 13 herum in Richtung zu dem Injektorkörper 7 hin kann der Faltkeil 2 in die Faltkammer 8 eingebracht werden und dadurch die Intraokularlinse 9 gefaltet werden. Die Bewegung des Halters 12 zum Einführen des Faltkeils 2 in die Faltkammer 8 ist eine Bewegung entlang eines Kreisbogens, wobei jedoch die Einführrichtung 26 als letzter Abschnitt der Bewegung in Figuren 1, 3 und 26 durch einen geraden Pfeil angedeutet ist.

Beispielsweise Figur 1 zeigt, dass die Injektoranordnung 1 einen Adapter 17 aufweisen kann, der eingerichtet ist, mit einem Kolben gekoppelt zu werden, in dem ein Stempel längsverschiebbar gelagert ist. Es ist denkbar, dass ein ophthalmochirurgischer Injektor geschaffen wird, indem die Injektoranordnung 1 mittels des Adapters 17 mit dem Kolben gekoppelt wird. Der ophthalmochirurgische Injektor weist somit die Injektoranordnung 1, den Kolben und den Stempel auf. Der Stempel kann eingerichtet sein, via den Adapter 17 in die Faltkammer 8 eingeführt zu werden und die von dem Faltkeil 2 gefaltete Intraokularlinse 9 aus der Faltkammer 8 heraus in eine Kanüle 14 der Injektoranordnung 1 zu schieben und von der Kanüle 14 aus der Injektoranordnung 1 heraus zu schieben.

Insbesondere Figuren 1 und 2 zeigen, dass der Faltkeil 2 ein feststehendes Faltkeilteil 20, das an dem Halter 12 befestigt ist, und ein bewegliches Faltkeilteil 19 aufweist, das beweglich an dem feststehenden Faltkeilteil 20 angebracht ist und von dem feststehenden Faltkeilteil 20 vorstehen kann. Das feststehende Faltkeilteil 20 kann einen Schlitz aufweisen, in dem das bewegliche Faltkeilteil 19 angeordnet ist. Durch die Anordnung mit dem feststehenden Faltkeilteil 20 und dem beweglichen Faltkeilteil 19 kann unterbunden werden, dass die Intraokularlinse 9 seitlich in Richtung zu dem Halter 12 hin wegrutscht und verklemmt.

Figuren 1 bis 6 zeigen eine erste Ausführungsform für die Injektoranordnung 1. Bei der ersten Ausführungsform ist der Anschlag von einem Steg 21, 22 gebildet, der in der Einführrichtung 26 gesehen in der Ausgangsposition mit der Faltkammeröffnung 18 überlappt. Das Bauteil ist zumindest teilweise von dem Faltkeil 2 gebildet oder vollständig von dem Faltkeil 2 gebildet. Es können beide Gleiter 3, 4 jeweils einen Anschlag haben. Dazu kann der erste Gleiter 3 einen ersten Steg 21 aufweisen und der zweite Gleiter 4 einen zweiten Steg 22 aufweisen, wie es auch in Figuren 1 bis 6 dargestellt ist. Zudem ist denkbar, dass der erste Gleiter 3 einen ersten Arm 23 aufweist, der von dem ersten Gleiter 3 absteht, wobei der erste Steg 21 von dem ersten Arm 23 absteht. Der erste Arm 23 kann dabei in einer ersten Bewegungsrichtung 15, in der der erste Gleiter 3 von der Ausgangsposition in die Endposition zu verschieben ist, von dem ersten Gleiter 3 vorstehen. Analog ist denkbar, dass der zweite Gleiter 4 einen zweiten Arm 24 aufweist, der von dem zweiten Gleiter 4 absteht, wobei der zweite Steg 22 von dem zweiten Arm 24 absteht. Der zweite Arm 24 kann dabei in einer zweiten Bewegungsrichtung 16, in der der zweite Gleiter 4 von der Ausgangsposition in die Endposition zu verschieben ist, von dem zweiten Gleiter 4 vorstehen. Der erste Arm 23 und/oder der zweite Arm 24 können in der Einführrichtung 26 gesehen vollständig außerhalb der Faltkammeröffnung 18 angeordnet sein. Der erste Steg 21 und/oder der zweite Steg 22 können vollständig außerhalb der Faltkammer 8 angeordnet sein.

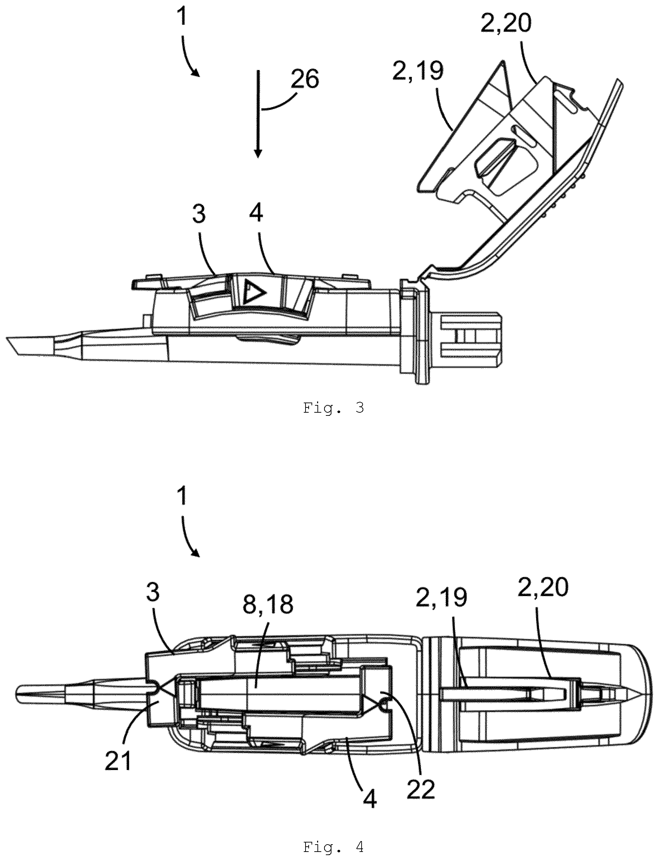

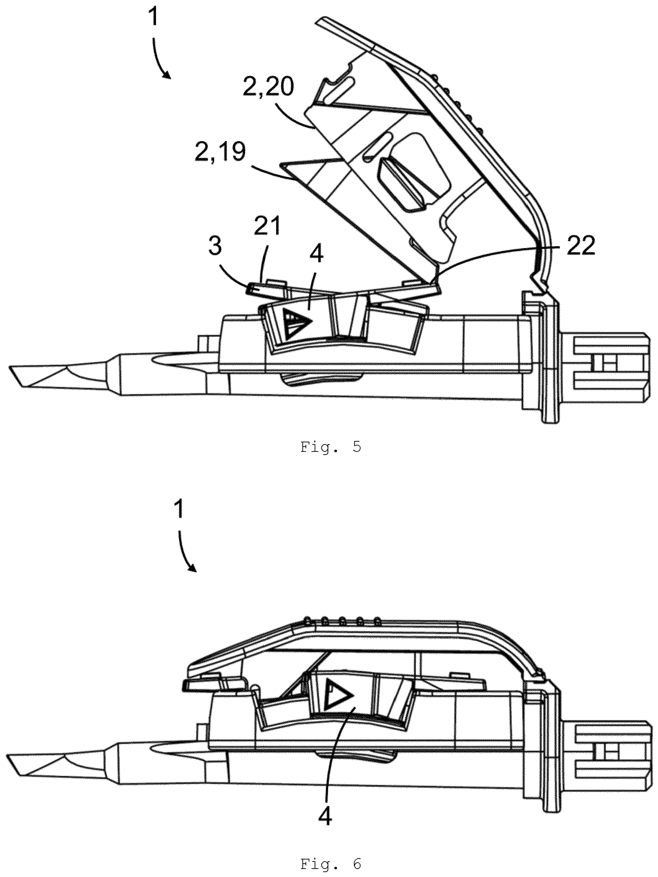

In Figuren 1 bis 6 ist die Funktionsweise der Injektoranordnung 1 beispielhaft für die erste Ausführungsform illustriert. In Figur 1 ist eine Seitenansicht und in Figur 2 ist eine Draufsicht der Injektoranordnung 1 dargestellt, wobei die Gleiter 3, 4 in der Ausgangsposition angeordnet sind und der Faltkeil 2 entfernt von den Gleitern 3, 4 angeordnet ist. In Figur 3 ist eine Seitenansicht und in Figur 4 ist eine Draufsicht der Injektoranordnung 1 dargestellt, wobei die Gleiter 3, 4 in der Endposition angeordnet sind und der Faltkeil 2 entfernt von den Gleitern 3, 4 angeordnet ist. In Figur 5 ist eine Seitenansicht der Injektoranordnung 1 dargestellt, wobei die Gleiter 3, 4 in der Ausgangsposition angeordnet sind und der Faltkeil 2 ausgehend von Figur 1 in Richtung zu der Faltkammer 8 hin bewegt wurde. Dabei stößt der Faltkeil 2 an den zweiten Steg 22 an. In dem Fall, dass der erste Gleiter 3 in der Ausgangsposition angeordnet ist und der zweite Gleiter 4 in der Endposition angeordnet ist, würde der Faltkeil 2 an den ersten Steg 21 anstoßen. In Figur 6 ist eine Seitenansicht der Injektoranordnung 1 dargestellt, wobei die Gleiter 3, 4 in der Endposition angeordnet sind. Hier kann der Faltkeil 2 in die Faltkammer 8 eingebracht werden, wie es auch dargestellt ist.

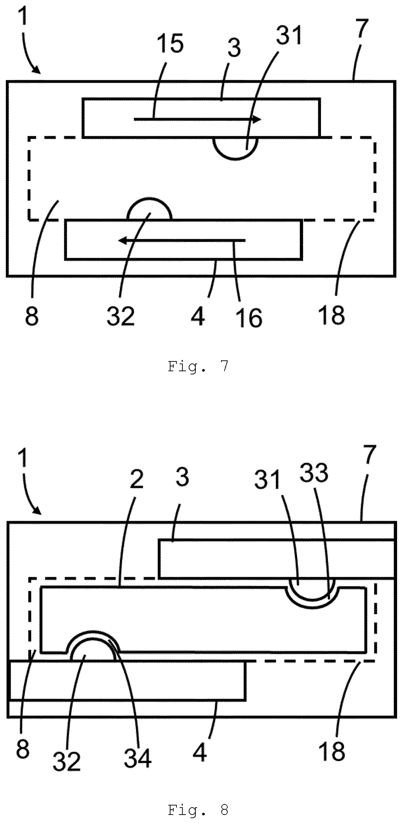

In Figuren 7 und 8 ist eine zweite Ausführungsform für die Injektoranordnung 1 dargestellt, wobei der Anschlag von einem in Richtung zu dem anderen Gleiter 3, 4 hin von dem Gleiter 3, 4 abstehenden Gleitervorsprung 31, 32 des Gleiters 3, 4 gebildet ist und der Faltkeil 2 eine Faltkeilaussparung 33, 34 aufweist, wobei der Gleitervorsprung 31, 32 eingerichtet ist, wenn in der Endposition der Faltkeil 2 in die Faltkammer 8 eingebracht wird, in der Faltkeilaussparung 33, 34 angeordnet zu sein, wodurch die Bewegung des Faltkeils 2 erlaubt ist. Das Bauteil ist zumindest teilweise von dem Faltkeil 2 gebildet oder vollständig von dem Faltkeil 2 gebildet. Es können beide Gleiter 3, 4 jeweils einen Anschlag haben. Dazu kann der erste Gleiter 3 einen ersten Gleitervorsprung 31 aufweisen, der zweite Gleiter 4 kann einen zweiten Gleitervorsprung 32 aufweisen, der Faltkeil 2 kann eine erste Falkteilaussparung 33 und eine zweite Faltkeilaussparung 34 aufweisen, wie es auch in Figuren 7 und 8 dargestellt ist. Dabei ist der erste Gleitervorsprung 31 eingerichtet, mit der ersten Faltkeilaussparung 33 in Eingriff zu stehen, und der zweite Gleitervorsprung 32 ist eingerichtet, mit der zweiten Faltkeilaussparung 34 in Eingriff zu stehen. In Figur 7 ist die Injektoranordnung 1 mit den Gleitern 3, 4 in der Ausgangsposition und ohne den Faltkeil 2 dargestellt, und in Figur 8 ist die Injektoranordnung 1 mit den Gleitern 3, 4 in der Endposition und mit dem in die Faltkammer 8 eingebrachten Faltkeil 2 dargestellt.

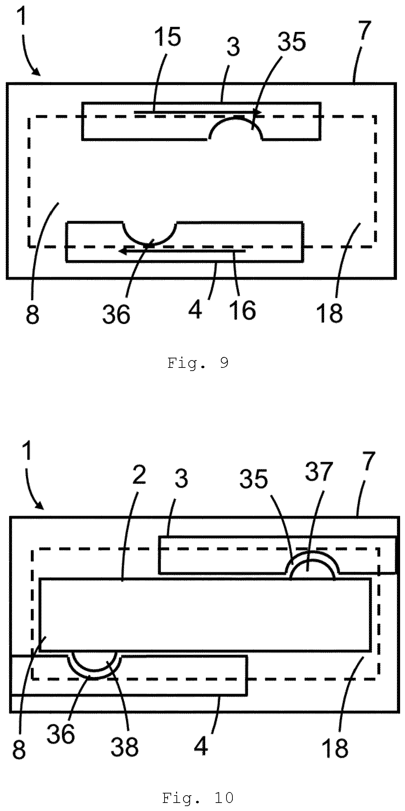

In Figuren 9 und 10 ist eine dritte Ausführungsform für die Injektoranordnung 1 dargestellt, wobei der Anschlag von dem Gleiter 3, 4 gebildet ist, der eine Gleiteraussparung 35, 36 aufweist, die in einer dem anderen Gleiter 3, 4 zugewandten Seite des Gleiters 3, 4 eingebracht ist, und der Faltkeil 2 einen Faltkeilvorsprung 37, 38 aufweist, der das Bauteil bildet und eingerichtet ist, wenn in der Endposition der Faltkeil 2 in die Faltkammer 8 eingebracht wird, in der Gleiteraussparung 35, 36 angeordnet zu sein, wodurch die Bewegung des Faltkeils 2 erlaubt ist. Das Bauteil ist zumindest teilweise von dem Faltkeil 2 gebildet oder vollständig von dem Faltkeil 2 gebildet. Es können beide Gleiter 3, 4 als jeweils ein Anschlag ausgebildet sein, wie es auch in Figuren 9 und 10 dargestellt ist. Dazu kann der erste Gleiter 3 eine erste Gleiteraussparung 35 aufweisen, der zweite Gleiter 4 eine zweite Gleiteraussparung 36 aufweisen und der Faltkeil 2 kann einen ersten Faltkeilvorsprung 37 und einen zweiten Faltkeilvorsprung 38 aufweisen. Dabei ist der erste Faltkeilvorsprung 37 eingerichtet, mit der ersten Gleiteraussparung 35 in Eingriff zu stehen, und der zweite Faltkeilvorsprung 38 ist eingerichtet, mit der zweiten Gleiteraussparung 36 in Eingriff zu stehen. In Figur 9 ist die Injektoranordnung 1 mit den Gleitern 3, 4 in der Ausgangsposition und ohne den Faltkeil 2 dargestellt und in Figur 10 ist die Injektoranordnung 1 mit den Gleitern 3, 4 in der Endposition und mit dem in die Faltkammer 8 eingebrachten Faltkeil 2 dargestellt.

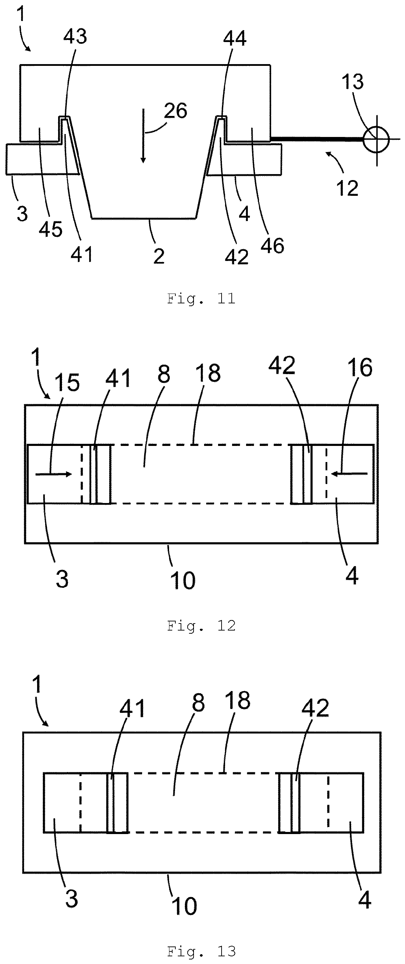

In Figuren 11 bis 13 ist eine vierte Ausführungsform für die Injektoranordnung 1 dargestellt, wobei der Anschlag von einem in Richtung entgegen der Einführrichtung 26 von dem Gleiter 3, 4 vorstehenden Gleitervorsprung 41, 42 des Gleiters 3, 4 gebildet ist, die Injektoranordnung 1 einen an dem Halter 12 angebrachten Vorsprung 45, 46 aufweist und der Gleitervorsprung 41, 42 sowie der Faltkeil 2 eine Aussparung 43, 44 begrenzen, wobei der Gleitervorsprung 41, 42 eingerichtet ist, in der Ausgangsposition an den Vorsprung 45, 46 anzustoßen, wodurch das Bauteil von dem Gleitervorsprung 41, 42 gebildet ist, und, wenn in der Endposition der Faltkeil 2 in die Faltkammer 8 eingebracht wird, in der Aussparung 43, 44 angeordnet zu sein, wodurch die Bewegung des Faltkeils 2 erlaubt ist. Es können beide Gleiter 3, 4 als jeweils ein Anschlag ausgebildet sein, wie es auch in Figuren 11 bis 13 dargestellt ist. Dazu kann der erste Gleiter 3 einen ersten Gleitervorsprung 41 aufweisen, der zweite Gleiter 4 einen zweiten Gleitervorsprung 42 aufweisen und der Faltkeil 2 kann einen ersten Vorsprung 45 und einen zweiten Vorsprung 46 aufweisen. Der erste Vorsprung 45 und der Faltkeil 2 begrenzen eine erste Aussparung 43 und der zweite Vorsprung 45 und der Faltkeil 2 begrenzen eine zweite Aussparung 44. Dabei ist der erste Gleitervorsprung 41 eingerichtet, mit der ersten Aussparung 43 in Eingriff zu stehen, und der zweite Gleitervorsprung 42 ist eingerichtet, mit der zweiten Aussparung 44 in Eingriff zu stehen, wie es auch in Figur 11 dargestellt ist. Figuren 12 und 13 zeigen, dass eine erste Bewegungsrichtung 15, in der der erste Gleiter 3 von der Ausgangsposition in die Endposition zu bewegen ist, zu dem zweiten Gleiter 4 hin gerichtet ist. In dem Fall, dass beide Gleiter 3, 4 als jeweils ein Anschlag ausgebildet sind, ist zudem eine zweite Bewegungsrichtung 16, in der der zweite Gleiter 4 von der Ausgangsposition in die Endposition zu bewegen ist, zu dem ersten Gleiter 3 hin gerichtet.

Alternativ ist denkbar, dass der Gleitervorsprung 41, 42 eingerichtet ist, in der Ausgangsposition an den Faltkeil 2 anzustoßen, wodurch das Bauteil von dem Faltkeil 2 gebildet ist. In diesem Fall ist eine erste Bewegungsrichtung 15, in der der erste Gleiter 3 von der Ausgangsposition in die Endposition zu bewegen ist, von dem zweiten Gleiter 4 weg gerichtet. In dem Fall, dass beide Gleiter 3, 4 als jeweils ein Anschlag ausgebildet sind, ist zudem eine zweite Bewegungsrichtung 16, in der der zweite Gleiter 4 von der Ausgangsposition in die Endposition zu bewegen ist, von dem ersten Gleiter 3 weg gerichtet.

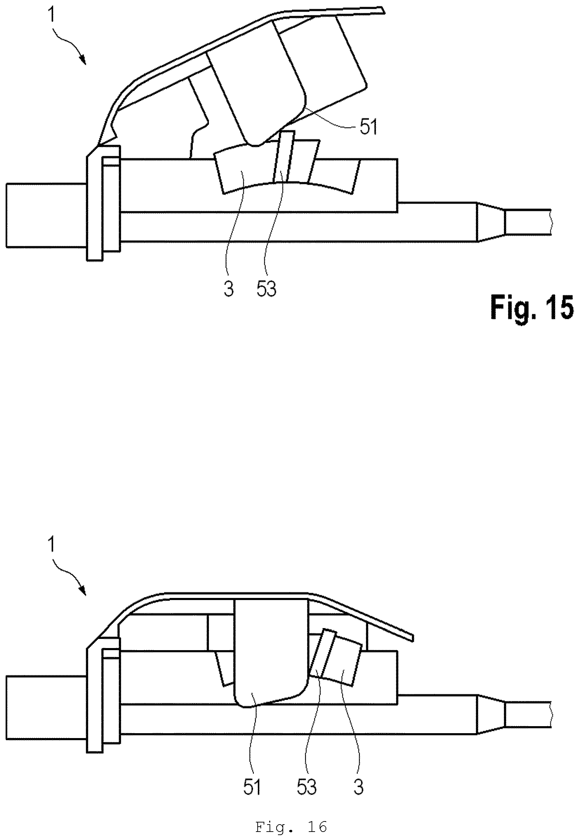

In Figuren 14 bis 16 ist eine fünfte Ausführungsform für die Injektoranordnung 1 dargestellt, wobei das Bauteil von einem an dem Halter 12 angebrachten Vorsprung 51, 52 gebildet ist, der von dem Faltkeil 2 räumlich getrennt ist. Der Anschlag ist von einem Gleitervorsprung 53, 54 des Gleiters 3, 4 gebildet. Es können beide Gleiter 3, 4 als jeweils ein Anschlag ausgebildet sein, wie es auch in Figuren 11 bis 13 dargestellt ist. Dazu kann der erste Gleiter 3 einen ersten Gleitervorsprung 53 aufweisen, der zweite Gleiter 4 kann einen zweiten Gleitervorsprung 54 aufweisen und die Injektoranordnung 1 kann einen ersten Vorsprung 51 und einen zweiten Vorsprung 52 aufweisen, die jeweils an dem Halter 12 angebracht sind. Der erste Gleitervorsprung 53 kann an einer dem zweiten Gleiter 4 abgewandten Seite von dem ersten Gleiter 3 vorstehen. Der zweite Gleitervorsprung 54 kann an einer dem ersten Gleiter 3 abgewandten Seite von dem zweiten Gleiter 4 vorstehen. Der erste Gleitervorsprung 53 ist eingerichtet, in der Ausgangsposition an den ersten Vorsprung 51 anzustoßen, und der zweite Gleitervorsprung 54 ist eingerichtet, in der Ausgangsposition an den zweiten Vorsprung 52 anzustoßen. Dies ist in Figur 15 dargestellt. Durch Schwenken des Halters 12 kann der Vorsprung 51 an den ersten Gleitervorsprung 53 und der Vorsprung 52 an den zweiten Gleitervorsprung 54 anstoßen. Dadurch ist es möglich, den ersten Gleiter 3 von einer Ausgangsposition in eine Endposition zu verlagern. Gleichzeitig ist es dadurch möglich, den zweiten Gleiter 4 von einer Ausgangsposition in die Endposition zu verlagern. In Figur 16 ist dargestellt, dass die Gleiter 3, 4 in der Endposition sind und der Faltkeil 2 in die Faltkammer 8 eingebracht ist. In Figur 14 sind die Gleiter 3, 4 sowohl in der Ausgangsposition als auch in der Endposition (mit gestrichelten Linien gezeichnet) dargestellt. Die Gleiter in der Ausgangsposition haben dabei die Bezugsbezugszeichen 3 und 4 und die zugehörigen Gleitervorsprünge die Bezugszeichen 53 und 54. Die Gleiter in der Endposition haben dabei die Bezugszeichen 3a und 4a und die zugehörigen Gleitervorsprünge die Bezugszeichen 53a und 54a.

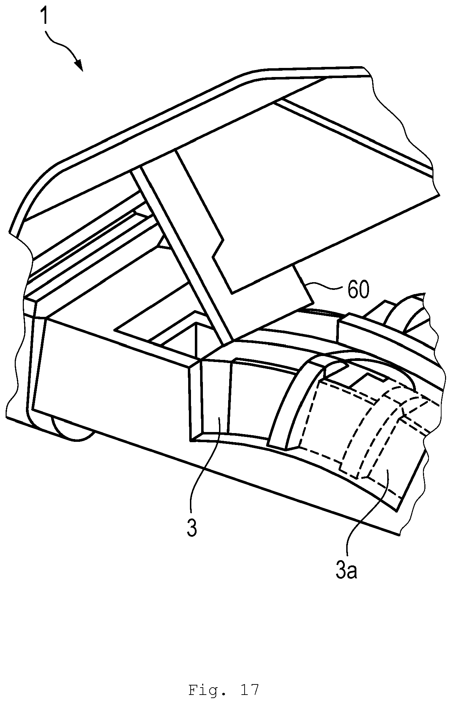

In Figur 17 ist eine sechste Ausführungsform für die Injektoranordnung 1 dargestellt, wobei das Bauteil von einem an dem Halter 12 angebrachten Vorsprung 60 gebildet ist, der von dem Faltkeil 2 räumlich getrennt ist und der Anschlag von einem der zwei Gleiter 3 gebildet ist. Der Gleiter ist dabei sowohl in seiner mit dem Bezugszeichen 3 bezeichneten Ausgangsposition als auch in seiner mit dem Bezugszeichen 3a bezeichneten Endposition (mit gestrichelten Linien gezeichnet) dargestellt. Der Vorsprung 60 darf nach einem Schwenken des Halters 12 nur so weit in die Injektoranordnung 1 eindringen, dass ein Stößel zum Befördern einer Intraokularlinse 9 in Richtung zur Kanüle 14 nicht behindert wird.

### Bezugszeichenliste

1 Injektoranordnung
2 Faltkeil
3 erster Gleiter
4 zweiter Gleiter
5 erste Gleiterbahn
6 zweite Gleiterbahn
7 Injektorkörper
8 Faltkammer
9 Intraokularlinse
10 Optikkörper
11 Haptik
12 Halter
13 Gelenk
14 Kanüle
15 erste Bewegungsrichtung
16 zweite Bewegungsrichtung
17 Adapter
18 Faltkammeröffnung
19 bewegliches Faltkeilteil
20 feststehendes Faltkeilteil
21 erster Steg
22 zweiter Steg
23 erster Arm
24 zweiter Arm
25 Gleiterzapfen
26 Einführrichtung
31 erster Gleitervorsprung
32 zweiter Gleitervorsprung
33 erste Faltkeilaussparung
34 zweite Faltkeilaussparung
35 erste Gleiteraussparung
36 zweite Gleiteraussparung
37 erster Faltkeilvorsprung
38 zweiter Faltkeilvorsprung
41 erster Gleitervorsprung
42 zweiter Gleitervorsprung
43 erste Aussparung
44 zweite Aussparung
45 erster Vorsprung
46 zweiter Vorsprung
51 erster Vorsprung
52 zweiter Vorsprung
53 erster Gleitervorsprung
54 zweiter Gleitervorsprung
60 Vorsprung

## Patentansprüche

1. Injektoranordnung mit einer Faltkammer (8), welche eingerichtet ist, eine Intraokularlinse (9), die einen Optikkörper (10) und zwei Haptiken (11) aufweist, aufzunehmen, einem Halter (12), einem Faltkeil (2), der an dem Halter (12) angebracht ist und eingerichtet ist, in einer Einführrichtung (26) via eine Faltkammeröffnung (18) in die Faltkammer (8) eingebracht zu werden und dadurch die Intraokularlinse (9) zu falten, und zwei Gleitern (3, 4), die jeweils eingerichtet sind, durch ein Verschieben des jeweiligen Gleiters (3, 4) aus einer Ausgangsposition in eine Endposition eine der zwei Haptiken (11) auf den Optikkörper (10) zu verschieben, **dadurch gekennzeichnet, dass** mindestens einer der zwei Gleiter (3, 4) einen Anschlag aufweist, der eingerichtet ist, in der Ausgangsposition des mindestens einen Gleiters (3, 4) an ein an dem Halter (12) angebrachtes Bauteil anzustoßen, wodurch eine Bewegung des Bauteils blockiert ist und es dadurch dem Faltkeil (2) nicht erlaubt ist, die Intraokularlinse (9) zu falten, und in der Endposition des mindestens einen Gleiters (3, 4) die Bewegung des Bauteils zu erlauben und es dadurch dem Faltkeil (2) zu erlauben, die Intraokularlinse (9) zu falten.

2. Injektoranordnung gemäß Anspruch 1, wobei das Bauteil zumindest teilweise von dem Faltkeil (2) gebildet ist.

3. Injektoranordnung gemäß Anspruch 1 oder 2, wobei der Anschlag von einem Steg (21, 22) gebildet ist, der in der Einführrichtung (26) gesehen in der Ausgangsposition mit der Faltkammeröffnung (18) überlappt.

4. Injektoranordnung gemäß einem der Ansprüche 1 bis 3, wobei der Anschlag von einem in Richtung zu dem anderen Gleiter (3, 4) hin von dem Gleiter (3, 4) abstehenden Gleitervorsprung (31, 32) des Gleiters (3, 4) gebildet ist und der Faltkeil (2) eine Faltkeilaussparung (33, 34) aufweist, wobei der Gleitervorsprung (31, 32) eingerichtet ist, wenn in der Endposition der Faltkeil (2) in die Faltkammer (8) eingebracht wird, in der Faltkeilaussparung (33, 34) angeordnet zu sein, wodurch die Bewegung des Faltkeils (2) erlaubt ist.

5. Injektoranordnung gemäß einem der Ansprüche 1 bis 4, wobei der Anschlag von dem Gleiter (3, 4) gebildet ist, der eine Gleiteraussparung (35, 36) aufweist, die in einer dem anderen Gleiter (3, 4) zugewandten Seite des Gleiters (3, 4) eingebracht ist, und der Faltkeil (2) einen Faltkeilvorsprung (37, 38) aufweist, der das Bauteil bildet und eingerichtet ist, wenn in der Endposition der Faltkeil (2) in die Faltkammer (8) eingebracht wird, in der Gleiteraussparung (35, 36) angeordnet zu sein, wodurch die Bewegung des Faltkeils (2) erlaubt ist.

6. Injektoranordnung gemäß Anspruch 1 oder 2, wobei der Anschlag von einem in Richtung entgegen der Einführrichtung (26) von dem Gleiter (3, 4) vorstehenden Gleitervorsprung (41, 42) des Gleiters (3, 4) gebildet ist, die Injektoranordnung (1) einen an dem Halter (12) angebrachten Vorsprung (45, 46) aufweist und der Gleitervorsprung (41, 42) sowie der Faltkeil (2) eine Aussparung (43, 44) begrenzen, wobei der Gleitervorsprung (41, 42) eingerichtet ist, in der Ausgangsposition an den Faltkeil (2) anzustoßen, wodurch das Bauteil von dem Faltkeil (2) gebildet ist, oder an den Vorsprung (45, 46) anzustoßen, wodurch das Bauteil von dem Gleitervorsprung (41, 42) gebildet ist, und, wenn in der Endposition der Faltkeil (2) in die Faltkammer (8) eingebracht wird, in der Aussparung (43, 44) angeordnet zu sein, wodurch die Bewegung des Faltkeils (2) erlaubt ist.

7. Injektoranordnung gemäß Anspruch 1, wobei das Bauteil von einem an dem Halter (12) angebrachten Vorsprung (51, 52, 60) gebildet ist, der von dem Faltkeil (2) räumlich getrennt ist.

8. Injektoranordnung gemäß Anspruch 7, wobei der Anschlag von dem Gleiter (3, 4) gebildet ist.

9. Injektoranordnung gemäß Anspruch 8, wobei der Anschlag von einem Gleitervorsprung (53, 54) des Gleiters (3, 4) gebildet ist.

10. Ophthalmochirurgischer Injektor mit einer Injektoranordnung gemäß einem der Ansprüche 1 bis 9.

## Claims

1. Injector assembly having a folding chamber (8) which is designed to accommodate an intraocular lens (9) comprising an optics body (10) and two haptics (11), a holder (12), a folding wedge (2), which is attached to the holder (12) and is designed to be introduced in an insertion direction (26) into the folding chamber (8) via a folding chamber opening (18) and thereby to fold the intraocular lens (9), and two sliders (3, 4) which are each designed to displace one of the two haptics (11) onto the optics body (10) by displacement of the respective slider (3, 4) from a starting position into an end position, **characterized in that** at least one of the two sliders (3, 4) has a stop which is designed to butt against a component attached to the holder (12) in the starting position of the at least one slider (3, 4), as a result of which a movement of the component is blocked and the folding wedge (2) is thereby not permitted to fold the intraocular lens (9), and, in the end position of the at least one slider (3, 4), to permit the movement of the component and to thereby permit the folding wedge (2) to fold the intraocular lens (9).

2. Injector assembly according to Claim 1, wherein the component is at least partially formed by the folding wedge (2).

3. Injector assembly according to Claim 1 or 2, wherein the stop is formed by a web (21, 22) which, as seen in the insertion direction (26), overlaps with the folding chamber opening (18) in the starting position.

4. Injector assembly according to one of Claims 1 to 3, wherein the stop is formed by a slider protrusion (31, 32) of the slider (3, 4) protruding from the slider (3, 4) in the direction of the other slider (3, 4), and the folding wedge (2) has a folding wedge recess (33, 34), wherein when, in the end position, the folding wedge (2) is introduced into the folding chamber (8), the slider protrusion (31, 32) is designed to be arranged in the folding wedge recess (33, 34), as a result of which the movement of the folding wedge (2) is permitted.

5. Injector assembly according to one of Claims 1 to 4, wherein the stop is formed by the slider (3, 4) which has a slider recess (35, 36) which is introduced in a side of the slider (3, 4) that faces the other slider (3, 4), and the folding wedge (2) has a folding wedge protrusion (37, 38), which forms the component and when, in the end position, the folding wedge (2) is introduced into the folding chamber (8), is designed to be arranged in the slider recess (35, 36), as a result of which the movement of the folding wedge (2) is permitted.

6. Injector assembly according to Claim 1 or 2, wherein the stop is formed by a slider protrusion (41, 42) of the slider (3, 4) protruding from the slider (3, 4) in a direction counter to the insertion direction (26), the injector assembly (1) has a protrusion (45, 46) attached to the holder (12), and the slider protrusion (41, 42) and the folding wedge (2) delimit a recess (43, 44), wherein the slider protrusion (41, 42) is designed to butt against the folding wedge (2) in the starting position, as a result of which the component is formed by the folding wedge (2), or to butt against the protrusion (45, 46), as a result of which the component is formed by the slider protrusion (41, 42), and when, in the end position, the folding wedge (2) is introduced into the folding chamber (8), to be arranged in the recess (43, 44), as a result of which the movement of the folding wedge (2) is permitted.

7. Injector assembly according to Claim 1, wherein the component is formed by a protrusion (51, 52, 60) which is attached to the holder (12) and is spatially separated from the folding wedge (2).

8. Injector assembly according to Claim 7, wherein the stop is formed by the slider (3, 4).

9. Injector assembly according to Claim 8, wherein the stop is formed by a slider protrusion (53, 54) of the slider (3, 4).

10. Ophthalmosurgical injector having an injector assembly according to one of Claims 1 to 9.

## Revendications

1. Ensemble injecteur comprenant une chambre de pliage (8), qui est conçue pour recevoir une lentille intraoculaire (9) qui comporte un corps optique (10) et deux haptiques (11), un support (12), une cale de pliage (2) qui est fixée au support (12) et conçue pour être introduite dans la chambre de pliage (8) dans un sens d'insertion (26) par une ouverture de chambre de pliage (18) et plier ainsi la lentille intraoculaire (9), et deux curseurs (3, 4) qui sont chacun conçus pour faire coulisser l'un des deux haptiques (11) sur le corps optique (10) par coulissement du curseur respectif (3, 4) d'une position de départ à une position finale, **caractérisé en ce qu'**au moins un des deux curseurs (3, 4) comporte une butée qui est conçue pour venir en butée contre un composant fixé au support (12) dans la position de départ de l'au moins un curseur (3, 4), ce qui bloque le déplacement du composant et empêche ainsi la cale de pliage (2) de plier la lentille intraoculaire (9), et permettre le déplacement du composant dans la position finale de l'au moins un curseur (3, 4) et permettre ainsi à la cale de pliage (2) de plier la lentille intraoculaire (9) .

2. Ensemble injecteur selon la revendication 1, le composant étant formé au moins partiellement par la cale de pliage (2).

3. Ensemble injecteur selon la revendication 1 ou 2, la butée étant formée par une nervure (21, 22) qui, vue dans le sens d'insertion (26), chevauche l'ouverture de la chambre de pliage (18) dans la position de départ.

4. Ensemble injecteur selon l'une des revendications 1 à 3, la butée étant formée par une saillie de curseur (31, 32) du curseur (3, 4) qui fait saillie du curseur (3, 4) en direction de l'autre curseur (3, 4) et la cale de pliage (2) comportant un évidement de cale de pliage (33, 34), la saillie de curseur (31, 32) étant conçue pour être disposée dans l'évidement de cale de pliage (33, 34) lorsque la cale de pliage (2) est introduite dans la chambre de pliage (8) dans la position finale, ce qui permet le déplacement de la cale de pliage (2).

5. Ensemble injecteur selon l'une des revendications 1 à 4, la butée étant formée par le curseur (3, 4) qui comporte un évidement de curseur (35, 36) qui est ménagé dans un côté du curseur (3, 4) qui est dirigé vers l'autre curseur (3, 4), et la cale de pliage (2) comportant une saillie de cale de pliage (37, 38) qui forme le composant et qui est conçue pour être disposée dans l'évidement de curseur (35, 36) lorsque la cale de pliage (2) est introduite dans la chambre de pliage (8) dans la position finale, ce qui permet le déplacement de la cale de pliage (2).

6. Ensemble injecteur selon la revendication 1 ou 2, la butée étant formée par une saillie de curseur (41, 42) du curseur (3, 4) qui fait saillie du curseur (3, 4) dans le sens opposé au sens d'insertion (26), l'ensemble injecteur (1) comportant une saillie (45, 46) fixée au support (12) et la saillie de curseur (41, 42) ainsi que la cale de pliage (2) délimitant un évidement (43, 44), la saillie de curseur (41, 42) étant conçue pour venir en butée contre la cale de pliage (2) dans la position de départ de façon à former le composant à partir de la cale de pliage (2) ou pour venir en butée contre la saillie (45, 46) de façon à former le composant à partir de la saillie de curseur (41, 42) et pour être disposée dans l'évidement (43, 44) lorsque la cale de pliage (2) est introduite dans la chambre de pliage (8) dans la position finale, ce qui permet le déplacement de la cale de pliage (2).

7. Ensemble injecteur selon la revendication 1, le composant étant formé à partir d'une saillie (51, 52, 60) qui est fixée au support (12) et qui est séparée spatialement de la cale de pliage (2).

8. Ensemble injecteur selon la revendication 7, la butée étant formée par le curseur (3, 4).

9. Ensemble injecteur selon la revendication 8, la butée étant formée à partir d'une saillie de curseur (53, 54) du curseur (3, 4).

10. Injecteur chirurgical ophtalmique comprenant un ensemble injecteur selon l'une des revendications 1 à 9.
